# EUROPEAN PATENT SPECIFICATION

(11) **EP 3 609 555 B1**
(45) Date of publication and mention of the grant of the patent: **24.02.2021**
(21) Application number: 18720801.2
(22) Date of filing: 06.04.2018
(51) Int. Cl.: A61M 3/02

(54) **PROCESS FOR PRODUCTION OF PRE-FILLED ENEMAS**
VERFAHREN ZUR HERSTELLUNG VON VORGEFÜLLTEN EINLÄUFEN
PROCÉDÉ DE PRODUCTION D'ENÉMAS PRÉ-REMPLIS

(30) Priority: 10.04.2017 IT 201700039384
(43) Date of publication of application: 19.02.2020
(73) Proprietor: Brevetti Angela S.r.L., 36071 Arzignano (VI) (IT)
(72) Inventor: CONSOLARO, Roberto, 36071 ARZIGNANO (VI) (IT)
(74) Representative: De Filippis, Sara
(86) International application number: PCT/IT2018/050062
(87) International publication number: WO 2018/189761

(56) References cited:
- WO-A1-2008/117320
- WO-A2-01/66165
- US-A- 3 486 503
- US-A- 4 619 645
- US-A- 6 110 150
- Anonymous: "Blow fill seal", Wikipedia, the free encyclopedia , XP002781642, Retrieved from the Internet: URL:https://en.wikipedia.org/wiki/Blow_fil l_seal [retrieved on 2018-06-05]

## Description

This invention relates to an insert for packaging pre-filled enemas. In detail, the invention relates to an insert for packaging pre-filled enemas which allows the enema to be kept hermetically closed until the moment of actual use.

In the production of the pre-filled enemas of a predetermined medication, it is necessary to provide inside the packaging a dispensing instrument to apply to the enema only after its opening.

This forces the user to handle all the components contained in the packaging, whilst it would be better to preserve the sterility of the dispensing element until its use.

The aim of this invention is to eliminate this problem, so that the enema can be used by the operator at any time, with a single manoeuvre, without further manipulations.

US 4 619 645 (A) describes pre-filled enemas having a protection element. There is a prior art process for the production of syringes, under patent no.

WO2011001456. According to this process, which describes an application of the technique called BLOW FILL SEAL or BFS (describes in patent documents WO2007007178, WO2010143219 and EP2998097) the hermetic closing (or Seal phase) occurs by forming a cap or other closing element connected to the hollow body of the container and removable by means of an area with a thinned, snap-off, cross-section.

Pre-filled enemas are also known positioned inside a covering bag to keep them sterile until the moment of use.

However, the cap or the covering bag which cover the stem or the entire enema have the drawback of being costly during the production phase, as they require an additional consumption of plastic material.

Consequently, in the case of large-scale production, the consumption of plastic material for producing the enema, the dimensions of the applicator to sterilise and subsequently to store and control in the assembly lines, as well as their subsequent disposal, render the enema unsustainable from an environmental, as well as economic, point of view.

There is therefore the need for a closing insert for pre-filled enemas which allows the containment and the conservation of a substance, which is easy to open by a user, but which at the same time is protected from contamination.

The aim of the invention is to overcome the drawbacks of the prior art.

More specifically, the main aim of the invention is to provide an insert for the packaging of pre-filled enemas and an enema equipped with the insert which ensures that the enema has the possibility of being used by the operator at any time, with a single manoeuvre.

More specifically, the aim of the invention is to provide an insert for the packaging of pre-filled enemas and an enema equipped with the insert which avoids the contamination of the medication until the moment of use. Another aim of the invention is to devise an insert for the packaging of pre-filled enemas and an enema equipped with the insert which, whilst ensuring the achievement of the above-mentioned aims, can be quickly actuated.

A further aim of the invention is to provide an insert for the packaging of pre-filled enemas and an enema equipped with the insert which guarantees a more functional use for the operator.

The above-mentioned aims are achieved by an insert for the packaging of pre-filled enemas and an enema equipped with the insert and assembly according to the accompanying independent claims 1, 7 and 12, to which reference should be made.

Further detailed application features of the process according to the invention are described in the relative dependent claims.

Advantageously, the insert according to the invention allows an enema to be set up without any manipulation of the components making up the enema by the operator.

This allows, for example, an enema to be rendered totally sterile and to allow, consequently, a subsequent use under optimum safety conditions from the point of view of the hygiene and health of the persons involved.

Moreover, when the material allows it, it is possible to subject the pre-filled enema obtained in this way to a final sterilisation, that is to say, when the material is resistant to the temperatures required by the process.

Also advantageously, this results in a considerable reduction in the production cost of an enema with respect to the prior art, obviously if the other factors involved are the same.

Moreover, advantageously, the enema obtained with the insert according to the invention has functionality characteristics at least equivalent to the prior art enemas with respect to which it maintains, in any case, the above-mentioned advantages.

In detail, the invention relates to an insert for packaging pre-filled enemas equipped with a supplying end, comprising a first end for coupling with the container, a second end for dispensing, and removable protection means, at the supplying end.

The removable protection means comprise an area of connection with the supplying end with a reduced cross-section with respect to the cross-section of the supplying end, for easy removal of the protection means.

This easy breakage (snap-off) area can, for example, comprise a portion with a reduced cross-section, with respect to the cross-section of the insert at the second end. An embodiment of the invention comprises the presence of a partial incision in the snap-off area, more precisely, along the base of the protection means.

In detail, the removable protection means can comprise a pin located at and protruding from the supplying end of the insert.

In any case, the removable protection means can comprise an improved adherence zone, for example grooves, preferably vertical, to increase the contact surface at least in a particular gripping area, in such a way as to facilitate their removal when desired.

The coupling end has a cavity designed to couple with the supplying end of the container; moreover, the coupling end can have first guide means for guiding the movement of the coupling end from a first position, close to the supplying end of the container, to a second position, wherein the insert is removed from the container.

The element can comprise a protection element, designed to protect the entire volume of the insert.

In that case, the coupling end may also have a convex part equipped with second guide means for guiding the movement of the protection element, from a first position, wherein the protection element is rigidly coupled to the insert, in such a way as to prevent accidental impacts to the removable protection means, to a second position, wherein the protection element is removed from the insert.

Moreover, the improved adherence zone of the protection means can comprise a rough surface, designed to increase the adherence between the protection means and a portion of protection element when applied to the insert, during packaging of the pre-filled enema.

This advantageously allows the closing of the pre-filled enema when the protection element is still hot and therefore malleable, simply by squashing a portion (for example the upper portion) of the protection element on the protection means in such a way as to incorporate them. The rough surface increases the adherence between the protection means and the portion of the protection element, in such a way that these become integral with each other.

In fact, by quashing the portion of the protection element when this still hot and malleable, the inner surface of the portion of the protection element is shaped on the rough surface, obtaining a counter-surface corresponding to the rough surface.

This allows, advantageously, rendering the rough surface of the protection means and the rough counter-surface of the inside of the protection element engaged with each other and integral in a removal movement.

In other words, with the same movement for removal of the protection element, the removable protection means will also be removed, since they are engaged by means of the respective rough surfaces.

The portion of the protection element has, at the removable protection means, gripping means for actuating the movement of the protection element from its first above-mentioned position to its above-mentioned second position, in which it is removed from the insert.

Preferably, the gripping means incorporate rigidly the removable protection means in such a way that, when the protective element passes from its first to its second position, the protection means are also removed from the insert, thereby allowing the immediate opening of the enema, rendering the enema directly available for use.

This allows the pre-filled enema to be kept sealed up to the actual moment of use, preventing the liquid entering the container from being contaminated during the intermediate opening and assembly steps.

At the same time, advantageously, the insert according to the invention allows the pre-filled container to be rendered immediately ready for use, without the operator being forced to handle sterile elements before time.

Preferably, according to the invention, the gripping means can comprises protruding elements designed to rotate in such a way as to actuate the movement of the protection element along the second guide means.

In fact, in this way, it is possible, advantageously, with a single fixed gesture to remove the protection element from the pre-filled enema and open the latter with the removal of the protection means from the insert, rendering the operation intuitive for anyone.

In detail, the first means for guiding the coupling end of the insert can comprise a first thread, which corresponds with a first counter-thread, present on the outer surface of the supplying end of the container and corresponding to the first thread.

Advantageously, this shape is easy to achieve during the process for forming the system, moulding the counter-thread using the pressure of the connection end on the enema when it is still hot, in order to shorten the production times of the insert.

The second guide means of the coupling element can also comprise a second thread, which corresponds to a second counter-thread, present on the outer surface of the protection element and corresponding to the second thread.

Advantageously, these guide means are easy to make, during the production process, according to the principle described above; moreover, the guide means are also very easy to use by anyone, rendering even more immediate the operation for opening the pre-filled container.

Similarly, the gripping means can also be made by pressing the protection element of the insert when the plastic of the protection element is still hot.

In this way, it is sufficient for the operator to unscrew the protection element (for example, a cap) in order to activate the movement of the guide means along the insert, thereby separating the protection means from the insert: once the protection means are removed, the pre-filled container is ready to dispense the liquid contained inside it.

In detail, the rough surface may be advantageously located at the base of the removable protection means, that is, close to the snap-off area.

The invention also relates to a pre-filled container for the dispensing of liquid, for example medical, which comprises an insert for packaging enemas according to the invention.

A further object of the invention is a process, for example according to the BFS technique, for making a pre-filled enema equipped with a supplying end, comprising a protection element designed to protect the supplying end, and which comprises the following steps:
- providing an insert according to the invention,
- coupling the first end of the insert with the supplying end,
- coupling the protection element to the insert, in such a way as to cover the entire volume of the insert.

This advantageously allows a pre-filled enema to be obtained which is fully packaged and directly ready for use once opened.

Preferably, according to the invention, the coupling of the insert with at least one between the enema and the protection element, occurs respectively when the enema and/or the protection element is still hot and therefore malleable.

This enables the production operations to be speeded up, obtaining the shaping of the guide means and/or of improved adherence surfaces simply by moulding given the pressure of the insert on the plastic still hot and therefore malleable.

Moreover, according to the invention, it is possible to incorporate in the protection element at least a portion of the protection means when the protection element is still hot.

This advantageously allows the protection element to be rendered integral with the protection means of the insert in an extremely short time, and without other intermediate operations, simply by exploiting the temporary malleability of the material from which the protection element is made.

The above-mentioned aims and advantages, as well as others described below, will become clearer from the following description relative to a preferred embodiment of the insert according to the invention, given by way of example and without limiting its scope, with reference to the accompanying drawings, in which:
Figure 1 illustrates a first exploded view of the closing insert according to the invention;
Figure 2 illustrates the exploded view of the insert of Figure 1 in a first position, applied to a portion of the enema and protected by a cap;
Figure 3 illustrates the exploded view of the insert of Figure 1 in a second position, applied to a portion of the enema and with the cap removed;
Figure 4 illustrates the enema with the insert of Figure 1 applied in a first closing position;
Figure 5 illustrates the enema of Figure 4, in a second intermediate position, during an opening movement;
Figure 6 illustrates the enema of Figure 4 in a third opening position;
Figure 7 illustrates a perspective view of the enema of Figure 6;
Figure 8 illustrates a view from above of the enema of Figure 4.

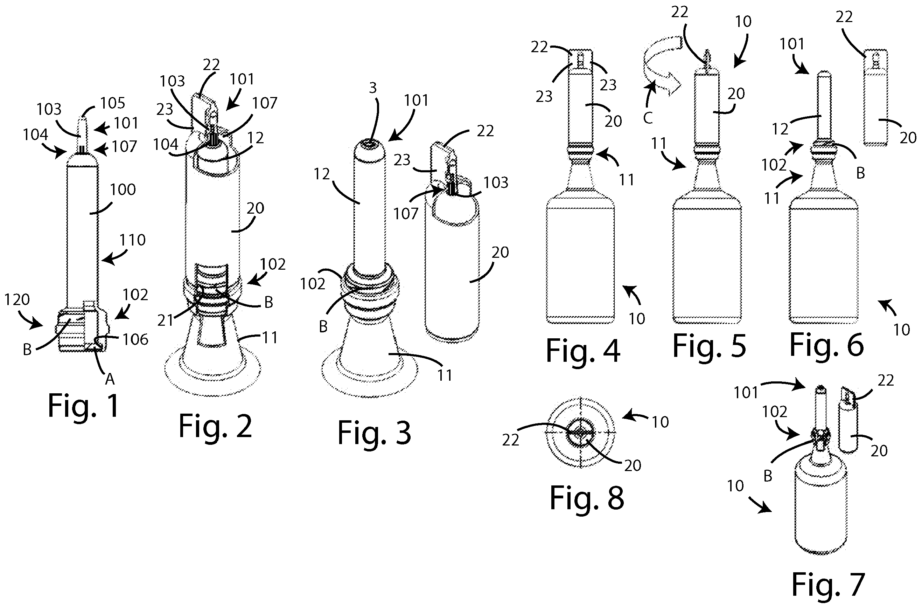

With reference to Figures 1 to 8, the closing insert 100 for pre-filled enemas 10 according to the invention comprise a first supplying end 101, and a second end 102 for picking up medical liquid and coupling with a container 10 in turn equipped with a supplying end 11 designed to be connected to the second end 102 for picking up medical liquid of the insert.

The first supplying end 101 has removal protection means 103, designed to prevent the escape of liquid from the container until the moment of actual use of the enema 10.

The removable protection means 103 are connected to the first supplying end 101 by a snap-off area 104, for example an area with reduced cross-section, or, in any case, weakened.

In the executive embodiment shown in the drawings, the removable protection means 103 are represented by a pin protruding from the supplying end 101 of the insert 100.

This advantageously allows an easy and immediate opening, simply levering on the free end 105 of the pin 103, with the pivot on the snap-off area 104.

The coupling end 102 may have a thread A on the inner wall 106, in such a way as to reversibly couple with supplying end 11 of the container 10.

The insert 100 can comprise a protection cap 20, designed to protect the entire volume of the insert 100.

In that case, the coupling end 102 may have an outer thread B, in such a way that the cap 20 can be formed by pressing its plastic when still hot, directly on the insert 100, so as to create a counter-thread 21 at the coupling end 102, and to incorporate the pin 103 at the supplying end 101. The protection cap 20 has, in any case, at the protection pin 103, gripping means 22 for actuating the movement of the protection cap 20 from its first coupling position (Figures 2, 4, 8) to its second position, in which it is removed from the insert 100 (Figures 3, 6, 7).

For example, the gripping means 22 are shown in a butterfly configuration. Substantially, the gripping means 22 incorporate rigidly the protection pin 103 in such a way that, when the protective cap 20 passes from its first to its second position, the pin 103, which is incorporated in the gripping means 22, is also removed from the insert, thereby allowing the immediate opening of the enema 10, rendering the enema 10 directly available for use.

It should be noted that, with regard to the accompanying drawings, the pin 103 is shown integral with the gripping means 22, which may also be integral with other portions of the cap 20, and the gripping means 22 may be in a position different from that indicated, without thereby adversely affecting the operation of the opening of the enema.

In any case, the gripping means 22 can comprise elements 23 protruding, for example radially, still designed to offer resistance to the rotation, in such a way as to actuate the movement of the cap 20 along the thread B, according to the arrow C.

Once the enema 10 is opened, this is ready to dispense the liquid using the insert 100, equipped with a special dispensing valve (not illustrated).

The invention also relates to a pre-filled container for the dispensing of liquid, for example an enema, which comprises an insert 100 for packaging enemas 10 according to the invention.

The supplying end 11 of the enema 10 may be made integral with the pick up end 102 of the insert after the step for introducing liquid inside the enema 10.

The cap 20, once formed on the insert 100, is rendered integral with the supplying end 11 of the enema 10 and, at the end 11, can have a snap-off zone to facilitate the removal of the cap 20 during the opening operation (Figure 5).

The pre-filled enema 10 may be made, for example, with the BFS technique.

Moreover, the cap 20 is coupled to the insert 100 incorporating the insert 100 between the cap 20 and the supplying end 11 of the container 10 when the container 10 and the cap 20 are still hot and therefore malleable. In other words, during the production of the pre-filled enema 10, the insert 100 after the step for filling the enema 10 to the relative supplying end, and the pin 103 is squashed and/or incorporated inside a portion 22 of the cap 20 (for example, the gripping means 22 are squashed on the rough surface 107) when the cap 20 is still hot and therefore malleable.

Advantageously, the rough surface 107 is positioned close to the snap-off area 104, at the base of the pin 103, increasing the surface of adherence with the portion 22 of the cap 20, thus improving the gripping between the cap 20 and the pin 103.

Similarly, the counter-thread 21 of the cap 20 is also formed when the plastic of the cap 20 is still hot and therefore malleable, again squashing the cap 20 with the mould on the insert 100 (as described in patent document no. EP2998097).

The rough surface 107 can be made comprising a series of grooves or a series of protruding elements, preferably with the main direction of extension parallel to the pin 103.

In particular, for example, the insert 100 has a substantially tubular shape, the cavity of which couples with the supplying end 11 of the enema 10 (for example, incorporates the supplying end of the pre-filled enema 10, in particular this may occur by coupling the insert 100 when the enema 10 is still hot and therefore malleable), whilst its outer surface has a thread B, corresponding to a counter-thread 21 present on the inner surface of the cap 20.

The insert 100, shown in Figure 1, has a hollow volume consisting substantially of a first hollow cylinder 110, provided with a first outer diameter, and preferably also a second hollow cylinder 120, provided with a second outer diameter, larger than the first.

The first cylinder 110 has a substantially anatomic shape for dispensing liquid contained in the enema 10.

The second hollow cylinder 120 acts as a spacer for the cap 20 in such a way as to prevent the cap 20 from adhering to the first cylinder 110 and it can be easily removed from it.

Moreover, preferably, the second hollow cylinder 120 has on its outer surface a thread B, which corresponds to a counter-thread 21 on the inner surface of the cap 20.

The gripping means 22 are rotated by the operator following the thread B present on the insert 100, as already described, and guide the cap 20 away from the supplying end 11 of the enema 10, whilst at the same time the cap 20 carries with it the pin 103.

Operatively, in any case, when the operator takes a container 10 of medical liquid to which the insert 100 according to the invention is applied, the insert 100 is in a first position, with the cap 20 and the pin 103 applied (Figures 2, 4, 8).

During opening, the operator acts on the cap 20, preferably directly on the tabs 23, rotating in a direction of rotation, for example according to direction C (Figure 5). In this way, the cap 20 starts to rotate and, as it is engaged with the pin 103 also in the rough surface 107, it detaches from the enema 10, removing also the pin 103 from the insert 100 (Figures 3, 6, 7).

In this way, the container 10 is free from the cap 20 and from the pin 103, ready to be used.

It can be understood from the above description that the insert according to the invention, the pre-filled enema and the process for its production and assembly, which are the object of the current invention, achieve the aims and reach the advantages mentioned above.

Modifications may be made to the insert according to the invention during the execution phases; in that case, the execution methods described above for the insert according to the invention will vary accordingly.

It should also be noted that the invention, whilst it has been described with particular reference to a disposal pre-filled enema, may also extend to other similar containers of liquids.

Where the constructional characteristics and the techniques mentioned in the following claims are followed by signs or reference numbers, the signs or reference numbers have been used only with the aim of increasing the intelligibility of the claims themselves and, consequently, they do not constitute in any way a limitation to the interpretation of each element identified, purely by way of example, by the signs or reference numbers.

## Claims

1. A process for making a pre-filled enema (10) equipped with a supplying end (11), comprising a protection element (20) designed to protect the supplying end (11),
comprising the following steps:
- providing an insert (100) comprising a first end (102) for the coupling with the enema (10), a second end (101) for the dispensing, and removable protection means (103), at the second end (101),
- coupling the first end (102) of the insert (100) with the supplying end (11),
- coupling the protection element (20) to the insert (100), in such a way as to cover the entire volume of the insert (100),
the process being **characterized in that** each of said steps is performed according to the Blow Fill Seal technique.

2. The process according to claim 1, **characterised in that** the coupling of the insert (100) with at least one between the enema (10) and the protection element (20), occurs respectively when the enema (10) and/or the protection element (20) is still hot.

3. The process according to claim 1 or 2, **characterised in that** it comprises the following step:
- incorporating in the protection element (20) at least a portion of the protection means (103) when the protection element (20) is still hot.

4. The process according to claim 1 or 2, **characterised in that** it comprises, before the step for coupling the first end (102) of the insert (100) with the supplying end (11), the following step:
- providing, between the removable protection means (103) and the supplying end (11), a snap-off connection area (104), for easy removal of the protection means (103).

5. The process according to claim 4, **characterised in that** the snap-off area comprises a portion (104) with reduced cross-section relative to the cross-section of the insert (100) a the second end (101).

6. The process according to any one of claims 1 to 5, **characterised in that** it comprises the following step:
- providing the protection means (103) with a pin protruding at the second end (101) of the insert (100).

7. The process according to any one of claims 1 to 6, **characterised in that** it comprises the following step:
- providing the protection means (103) with an improved adherence zone (107).

8. The process according to any one of claims 1 to 7, **characterised in that** it comprises the following step:
- providing, on the outer surface of the first end (102), first guide means (B) for guiding the movement of a protection element (20) from a position of coupling with the insert (100) to a removal position.

9. The process according to claim 8, **characterised in that** during the step of coupling the protection element (20) to the insert (100), in such a way as to cover the entire volume of the insert (100), second guide means (21) are created, corresponding to the first guide means (B) of the insert (100).

10. The process according to any one of claims 1 to 9, **characterised in that** the protection element (20) comprises gripping means (22, 23), which incorporate the removable protection means (103, 107).

11. A pre-filled enema (10) made according to the process of any one of claims 1 to 10.

## Patentansprüche

1. Verfahren zur Herstellung eines vorgefüllten Einlaufs (10), der mit einem Ausgabeende (11) ausgestattet ist, das ein Schutzelement (20) umfasst, das zum Schützen des Ausgabeendes (11) konstruiert ist,
umfassend folgende Schritte:
- Bereitstellen eines Einsatzes (100), der ein erstes Ende (102) zum Koppeln mit dem Einlauf (10), ein zweites Ende (101) zum Abgeben und abnehmbare Schutzmittel (103) an dem zweiten Ende (101) umfasst,
- Koppeln des ersten Endes (102) des Einsatzes (100) mit dem Ausgabeende (11),
- Koppeln des Schutzelements (20) mit dem Einsatz (100) so, dass das gesamte Volumen des Einsatzes (100) abgedeckt ist,
wobei das Verfahren **dadurch gekennzeichnet ist, dass** jeder der Schritte gemäß der Technik Blasen-Füllen-Versiegeln ausgeführt wird.

2. Verfahren nach Anspruch 1, **dadurch gekennzeichnet, dass** das Koppeln des Einsatzes (100) mit mindestens einem zwischen dem Einlauf (10) und dem Schutzelement (20) jeweils erfolgt, wenn der Einlauf (10) und/oder das Schutzelement (20) noch heiß ist.

3. Verfahren nach Anspruch 1 oder 2, **dadurch gekennzeichnet, dass** es folgenden Schritt umfasst:
- Integrieren von mindestens einem Abschnitt der Schutzmittel (103) in das Schutzelement (20), wenn das Schutzelement (20) noch heiß ist.

4. Verfahren nach Anspruch 1 oder 2, **dadurch gekennzeichnet, dass** es vor dem Schritt des Koppelns des ersten Endes (102) des Einsatzes (100) mit dem Ausgabeende (11) folgenden Schritt umfasst:
- Bereitstellen eines abtrennbaren Verbindungsbereichs (104) zum einfachen Abnehmen der Schutzmittel (103) zwischen den abnehmbaren Schutzmitteln (103) und dem Ausgabeende (11).

5. Verfahren nach Anspruch 4, **dadurch gekennzeichnet, dass** der abtrennbare Verbindungsbereich einen Abschnitt (104) mit im Verhältnis zum Querschnitt des Einsatzes (100) an dem zweiten Ende (101) verringertem Querschnitt umfasst.

6. Verfahren nach einem der Ansprüche 1 bis 5, **dadurch gekennzeichnet, dass** es folgenden Schritt umfasst:
- Bereitstellen der Schutzmittel (103) mit einem Stift, der an dem zweiten Ende (101) des Einsatzes (100) vorsteht.

7. Verfahren nach einem der Ansprüche 1 bis 6, **dadurch gekennzeichnet, dass** es folgenden Schritt umfasst:
- Bereitstellen der Schutzmittel (103) mit einer Zone (107) mit verbesserter Haftung.

8. Verfahren nach einem der Ansprüche 1 bis 7, **dadurch gekennzeichnet, dass** es folgenden Schritt umfasst:
- Bereitstellen an der Außenoberfläche des ersten Endes (102) von ersten Führungsmitteln (B) zum Führen der Bewegung eines Schutzelements (20) von einer Position des Koppelns mit dem Einsatz (100) zu einer Abnehmposition.

9. Verfahren nach Anspruch 8, **dadurch gekennzeichnet, dass** während des Schritts des Koppelns des Schutzelements (20) mit dem Einsatz (100) so, dass das gesamte Volumen des Einsatzes (100) abgedeckt ist, zweite Führungsmittel (21) erstellt werden, die den ersten Führungsmitteln (B) des Einsatzes (100) entsprechen.

10. Verfahren nach einem der Ansprüche 1 bis 9, **dadurch gekennzeichnet, dass** das Schutzelement (20) Greifmittel (22, 23) umfasst, in die die abnehmbaren Schutzmittel (103, 107) integriert sind.

11. Vorgefüllter Einlauf (10), hergestellt nach dem Verfahren nach einem der Ansprüche 1 bis 10.

## Revendications

1. Procédé de fabrication d'un énéma (10) pré-rempli équipé d'une extrémité d'alimentation (11), comprenant un élément de protection (20) conçu pour protéger l'extrémité d'alimentation (11),
comprenant les étapes suivantes :
- la fourniture d'un insert (100) comprenant une première extrémité (102) pour le couplage avec l'énéma (10), une seconde extrémité (101) pour la distribution, et un moyen de protection amovible (103), au niveau de la seconde extrémité (101),
- le couplage de la première extrémité (102) de l'insert (100) avec l'extrémité d'alimentation (11),
- le couplage de l'élément de protection (20) à l'insert (100), de manière à recouvrir le volume entier de l'insert (100),
le procédé étant **caractérisé en ce que** chacune desdites étapes est effectuée selon la technique de formage-remplissage-scellage.

2. Procédé selon la revendication 1, **caractérisé en ce que** le couplage de l'insert (100) avec au moins un parmi l'énéma (10) et l'élément de protection (20) se produit respectivement lorsque l'énéma (10) et/ou l'élément de protection (20) est toujours chaud.

3. Procédé selon la revendication 1 ou 2, **caractérisé en ce qu'**il comprend l'étape suivante :
- l'incorporation dans l'élément de protection (20) d'au moins une partie du moyen de protection (103) lorsque l'élément de protection (20) est toujours chaud.

4. Procédé selon la revendication 1 ou 2, **caractérisé en ce qu'**il comprend, avant l'étape pour le couplage de la première extrémité (102) de l'insert (100) avec l'extrémité d'alimentation (11), l'étape suivante :
- la fourniture, entre le moyen de protection amovible (103) et l'extrémité d'alimentation (11), d'une région de connexion par encliquetage (104), pour un retrait facile du moyen de protection (103).

5. Procédé selon la revendication 4, **caractérisé en ce que** la région d'encliquetage comprend une partie (104) avec une section transversale réduite par rapport à la section transversale de l'insert (100) au niveau de la seconde extrémité (101).

6. Procédé selon l'une quelconque des revendications 1 à 5, **caractérisé en ce qu'**il comprend l'étape suivante :
- le fait de doter le moyen de protection (103) d'une broche faisant saillie au niveau de la seconde extrémité (101) de l'insert (100).

7. Procédé selon l'une quelconque des revendications 1 à 6, **caractérisé en ce qu'**il comprend l'étape suivante :
- le fait de doter le moyen de protection (103) d'une zone d'adhérence améliorée (107).

8. Procédé selon l'une quelconque des revendications 1 à 7, **caractérisé en ce qu'**il comprend l'étape suivante :
- la fourniture, sur la surface externe de la première extrémité (102), d'un premier moyen de guidage (B) pour guider le mouvement d'un élément de protection (20) d'une position de couplage avec l'insert (100) à une position de retrait.

9. Procédé selon la revendication 8, **caractérisé en ce que** durant l'étape de couplage de l'élément de protection (20) à l'insert (100), de manière à recouvrir le volume entier de l'insert (100), un second moyen de guidage (21) est créé, correspondant au premier moyen de guidage (B) de l'insert (100).

10. Procédé selon l'une quelconque des revendications 1 à 9, **caractérisé en ce que** l'élément de protection (20) comprend un moyen de saisie (22, 23), qui incorpore le moyen de protection amovible (103, 107).

11. Énéma pré-rempli (10) fabriqué selon le procédé selon l'une quelconque des revendications 1 à 10.
